# EUROPEAN PATENT APPLICATION

(11) **EP 3 450 978 A1**
(43) Date of publication of application: **06.03.2019**
(21) Application number: 17789384.9
(22) Date of filing: 19.04.2017
(51) Int. Cl.: G01N 33/53, C12Q 1/04, G01N 33/543

(54) **INDEX OF LOCAL SEVERITY OF ATOPIC DERMATITIS IN SKIN AND THERAPEUTIC EFFECT THEREON**

(30) Priority: 28.04.2016 JP 2016092020
(71) Applicant: Shiseido Company Ltd., Chuo-ku Tokyo 104-0061 (JP)
(72) Inventor: TANAKA, Chika, Yokohama-shi Kanagawa 224-8558 (JP); MIZUMOTO, Chieko, Yokohama-shi Kanagawa 224-8558 (JP); ONODERA, Tomoko, Yokohama-shi Kanagawa 224-8558 (JP); AIBA, Setsuya, Sendai-shi Miyagi 980-8577 (JP); KIKUCHI, Katsuko, Sendai-shi Miyagi 980-8577 (JP); YAMASAKI, Kenshi, Sendai-shi Miyagi 980-8577 (JP); OZAWA, Maki, Sendai-shi Miyagi 980-8577 (JP)
(74) Representative: Scholz, Volker
(86) International application number: PCT/JP2017/015792
(87) International publication number: WO 2017/188101

(57) **Abstract**

Provided is an objective and convenient index for judging full recovery from atopic dermatitis and severity thereof. The present invention provides a diagnosis assisting method for determining a treatment policy for an atopic dermatitis subject under the treatment, said method comprising: measuring the expression value of SCCA-1 in skin horny cells of the subject; i) when the SCCA-1 expression value is not statistically significantly higher than the level of those who do not suffer from atopic dermatitis, then determining that the continuation of the treatment for the disease becomes unnecessary thanks to the treatment; and, in the case where the continuation of the treatment is determined as still necessary, ii) ranking the severity of atopic dermatitis of the subject using the SCCA-1 expression value as an index and determining an adequate treatment method for atopic dermatitis to be given to the subject depending on the rank.

## Description

### FIELD

The present invention provides a non-invasive method for evaluating local clinical conditions of atopic dermatitis by using squamous cell carcinoma antigen-1 (hereunder, referred to as "SCCA-1") in corneocytes as an indicator.

### BACKGROUND

Atopic dermatitis is one of the most common skin disorders. Although atopic dermatitis frequently occurs primarily during early childhood, recently the number of adolescent or older patients has been increasing. Atopic dermatitis is a disease that undergoes repeated exacerbation and remission and has eczema with pruritus as the primary etiology thereof. Although the mechanism of occurrence thereof is unclear, it is thought to occur based primarily on genetic factors in combination with various environmental factors. Genetic factors include 1) a family history or medical history of bronchial asthma, allergic rhinitis, conjunctivitis or atopic dermatitis, or 2) factors susceptible to production of IgE antibody (Japanese Dermatological Association, Guidelines for the Treatment of Atopic Dermatitis (NPL1)). Atopic dermatitis can present with a mixture of different severities of skin eruption even within the same patient, and the observation of fluctuations and repetition of exacerbation and remission dependent upon various factors during the course of the disease is also a characteristic thereof. For example, fluctuations in symptoms induced by the seasons, fluctuations caused by changes in the environment, or increasing severity or difficulty of treatment caused by various factors are observed. Treatment status also has a considerable effect on progress.

When treating atopic dermatitis, it is extremely important to control the symptoms and prevent exacerbation. Atopic dermatitis is a chronic disease for which it is difficult to achieve a complete cure in the true sense of the word. Consequently, the aim of treatment is to maintain an asymptomatic period of remission and prevent exacerbation in terms of appearance and the awareness of the patient. There has recently been suggested to be a correlation between atopic dermatitis and food and other allergies, and it is necessary to provide suitable treatment and approach remission as early as possible particular during early childhood. Since atopic dermatitis is a disease for which treatment frequently extends over a long period of time regardless of whether the onset thereof occurs during early childhood or adulthood, particular consideration should be given to an adequate explanation to patients, and treatment compliance and adherence.

At present, determination of the severity of atopic dermatitis is almost exclusively dependent on visual findings. Although there are various visual findings, such as drying symptoms, erythema, scales, papulation, excoriation, swelling, edema, crusting, small blisters, erosion or itchy nodules, since all of these incorporate subjective factors, there is the potential for discrepancies to occur in diagnosis depending on the skill of the physician, thereby resulting in the problem of diagnosis of the severity of atopic dermatitis lacking objectivity. The physician or other health care professional determines a treatment strategy for the patient while relying on a diagnosis of severity based on visual findings.

The Severity Scoring of Atopic Dermatitis (SCORAD) and Eczema Area and Severity Index (EASI) developed by the European Task Force on Atopic Dermatitis are frequently used worldwide to classify the severity of atopic dermatitis (NPL1). Both classify severity according to highly objective scores, with the highest score for SCORAD being 103 points and that for EASI being 72 points. In the case of SCORAD, which is used particularly frequently, scoring of skin symptoms requires the steps of: determining the total score for the degree of various factors such as the area of the eruption (ratio of eruption area to total body surface area), erythema, oozing/papulation, exudate/crusting, excoriation, lichenification or xerosis/dryness (0 = none, 1 = mild, 2 = moderate and 3 = severe); evaluating subjective symptoms by the subject according to the visual analogue scale (VAS); and finally calculating SCORAD score. Calculation of SCORAD score requires the specialized skills of an experienced physician while also having the shortcoming of requiring considerable time and effort. It is not desirable to require time in making an evaluation in the case the subject is an infant or child. Thus, there is a need for a method that enables severity to be classified easily and objectively. A severity determination method is advocated by the Japanese Society of Allergology that is less specialized and less complex than SCORAD (NPL2). Although this method comprises determining severity based on the ratio of eruption area to total body surface area, as mentioned above, since atopic dermatitis is a disease in which different degrees of severity are present within the same patient, this method is not considered to be adequate.

Although atopic dermatitis in the case of an eruption of mild severity can be treated with moisturizers, protective agents and skin care for the purpose of supplementing drying and decreases in skin barrier function as well as preventing recurrence of inflammation, as severity progresses, adequate effects cannot be obtained with the use of protective agents and the like alone. Topical steroid preparations and tacrolimus ointment (topical calcineurin inhibitor) are used to suppress inflammation associated with atopic dermatitis.

Cases of continuous use of large amounts of topical steroid preparations during routine examination and treatment have been reported to cause adverse side effects such as suppression of adrenal function in extreme cases. If topical steroid preparations are used properly at the amounts for usual routine treatment, systemic adverse side effects observed following administration of internal medications, such as adrenal insufficiency, diabetes or moon face, will not occur. Although local adverse side effects such as steroid acne, steroid flushing, atrophoderma, polytrichosis or bacterial, fungal or viral skin infections may occasionally occur, recovery from these conditions is achieved by discontinuing treatment or by taking appropriate actions. However, since topical steroid preparations are frequently used in combination with steroid internal medications and exacerbation of atopic dermatitis per se frequently occurs in conjunction with the occurrence of adverse side effects of topical steroid preparations, there are many aspects of topical steroid preparations that are misunderstood, resulting in fear and avoidance of topical steroid preparations on the patients side, and often leading to decreased compliance. Due to this misunderstanding, there are cases in which therapeutic effects are not obtained as a result of not applying the necessary topical steroid preparation. Accordingly, if it were possible to present the severity of the eruption to the patient, provide an adequate explanation of the meaning of the topical application of the corresponding steroid and have the patient consent to the application thereof, the selected topical application would be used properly and adequate therapeutic effects would be able to be achieved. For this reason as well, it is important to enhance the persuasiveness of treatment and produce adequate therapeutic effects by presenting the patient with the severity of the eruption based on an objective indicator of local skin severity.

It is necessary to select the strength and drug form of topical steroid preparations, which are classified in order to strength of clinical efficacy, in consideration of such factors as the severity of individual eruptions, application site or age. Since there is typically a balance between the strength of the effect of a topical steroid preparation and the likelihood of the occurrence of adverse side effects, it is important to not select a topical steroid preparation that is stronger than necessary, but rather suitably select a preparation that is ranked to match the individual eruption. For this reason as well, it is desirable to present an objective indicator of the local severity of atopic dermatitis.

In addition, as mentioned above, although cases of mild eruption severity can be accommodated with skin care using moisturizers or protective agents and the like that do not contain steroids or tacrolimus, it is still necessary to continue topical treatment even for mild skin conditions, and failure to do so may facilitate recurrence of inflammation making it necessary to use more potent topical steroid preparations. However, since determination of the severity of atopic dermatitis relies on visual findings as mentioned above, there are many cases in which unnecessary treatment is actually continued despite the absence of a need for medication and cases in which symptoms end up recurring as a result of discontinuing necessary treatment. The most common problem is that the patient assumes that atopic dermatitis has completely healed at his or her own discretion without seeking the advice of a physician, thereby resulting in the patient interrupting or completely discontinuing treatment. In particular, atopic dermatitis has a state of so-called "remission" in which disease is determined to be absent on the basis of external visual findings. For patients, "remission" means that the eruption is no longer present and there is no pruritus or other subjective symptoms, and a physician aims to maintain that "remission". However, even if eczema temporarily improves through the use of a topical anti-inflammatory preparation and leads to "remission", there are cases where perivascular lymphocytes which are low-level in terms of histology cause inflammatory cell infiltration, swelling of endothelial cells and mild lesions accompanying basement membrane thickening (Fiset, P.O., Leung, D. Y., JACI: 118:287-290, 2006) (NPL3). Since it is extremely difficult to determine skin conditions during remission based on external visual findings alone, in conventional treatments so-called "reactive therapy" have been employed in which medication is switched over to skin care using moisturizers and the like during remission, and once visibly recognizable inflammation has returned, medication is switched over to steroids and tacrolimus. However, if were possible to determine skin conditions during remission, proactive therapy (preventive intermittent application) could be employed in which treatment using a mild anti-inflammatory agents and skin care is provided prior to prominent recurrence of symptoms. For this reason as well, there is a strong desire for a subjective local indicator that makes it possible to determine skin conditions that are not easily recognized with the naked eye.

In this manner, despite the importance of controlling the symptoms of atopic dermatitis, it is difficult to determine skin conditions associated therewith, and further due to the adverse side effects of therapeutic drugs being used along with misunderstandings among patients regarding those adverse side effects, it is essential to provide patients with adequate explanations and consider patient compliance and adherence, and for these reasons as well, the providing of an objective indicator of severity is strongly desired by both patients and physicians.

Substances capable of serving as indicators of the severity of atopic dermatitis include peripheral blood neutrophil count, serum total IgE level, lactate dehydrogenase (LDH) level and serum thymus- and activation-regulated chemokine (TARC) level (Sugawara, et al., Allergy (2002), 57:180-181: NPL4). Serum TARC level correlates with the severity of atopic dermatitis, enabling it to serve as an indicator of systemic disease state. However, although atopic dermatitis may occur over the entire body in some cases, it frequently occurs locally and severity thereof often differs at each affected site in many cases. Thus, it is necessary to determine a treatment strategy that is compatible with the severity at each affected site, and an indicator that enables local diagnosis instead of a systemic indicator is required to diagnose atopic dermatitis. In addition, collection of blood samples for measuring TARC level in serum is invasive and lacks simplicity. In particular, since infants and small children account for roughly 89% of the incidence of atopic dermatitis, blood collection associated with pain places a burden on the patient and is technically difficult in the health care setting, thereby making it difficult to use blood to make an objective diagnosis of severity. Moreover, although an indicator that sensitively reflects disease progression is required since atopic dermatitis is exacerbated in a short period of time in cases of early childhood, existing indicators have the shortcoming of not reflecting disease progression with adequate sensitivity or demonstrating high values in cases of early childhood.

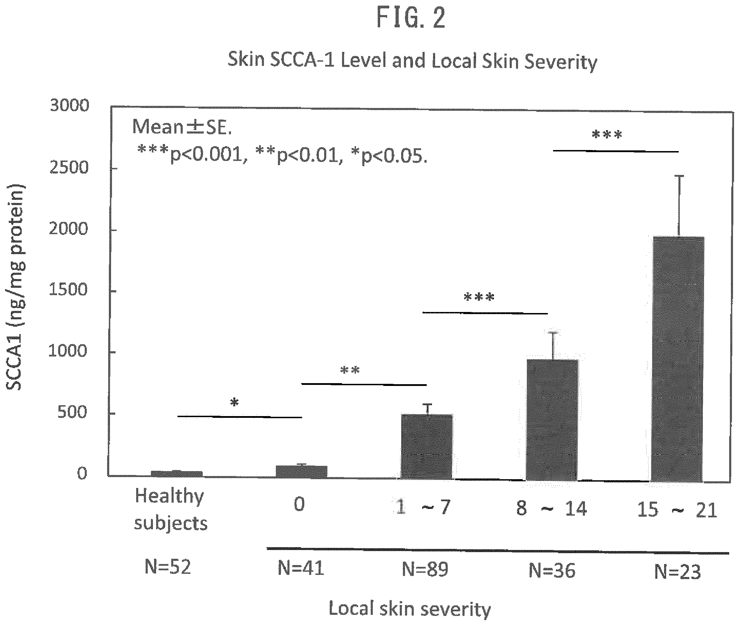

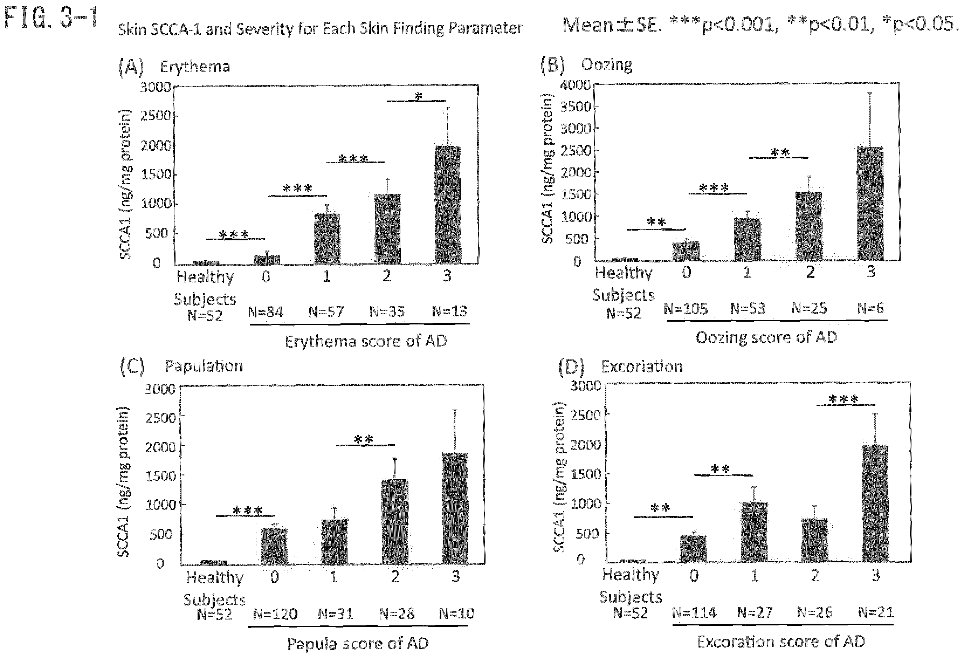

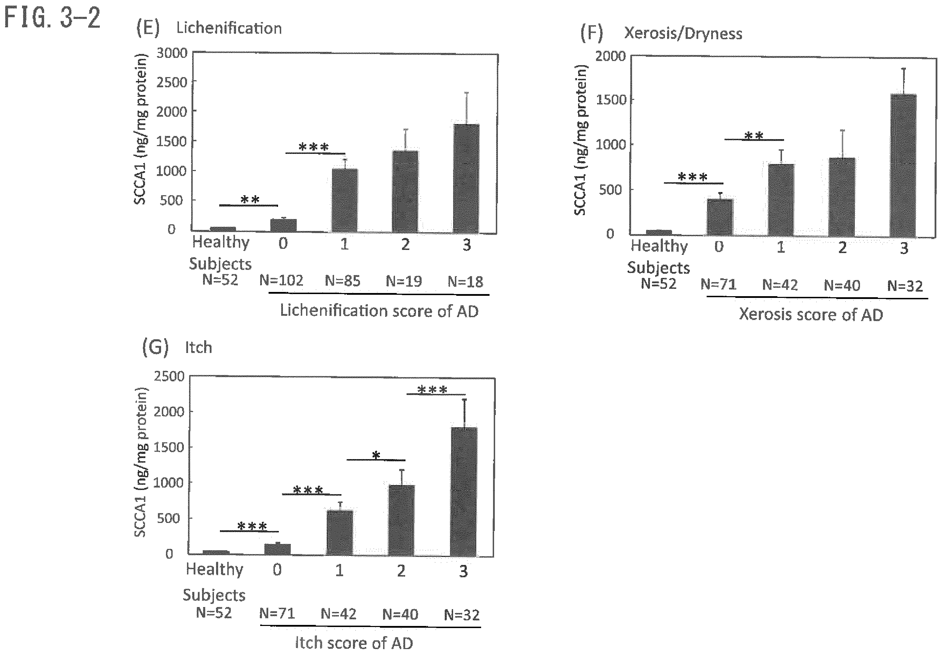

On the other hand, TARC present in the corneal layer (to be referred to as "corneal layer TARC") are inadequate as an indicator for objectively determining local severity. This is because a significant difference is not observed between measured values of corneal layer TARC in healthy subjects and levels of corneal layer TARC derived from eruption-free areas of atopic dermatitis patients (Morita, et al., Allergy (2010), 65:1166-1172: Fig. 2 of NPL5). Moreover, since significant differences in corneal layer TARC levels are not observed between patients exhibiting moderate severity and patients exhibiting severe symptoms (Fig. 3 of NPL4), corneal layer TARC level demonstrates a low correlation with severity. Thus, in the case of using TARC as an indicator of the severity of atopic dermatitis, there is no other choice than to measure serum TARC, which lacks simplicity from the viewpoint of collecting a serum sample, while also being unable to be used for local diagnosis, thereby preventing it from being regarded as an effective indicator. As mentioned above, since many atopic dermatitis patients are infants and small children, invasive sample collection in the manner of blood collection presents difficulties for both the patient and physician and is preferably avoided.

Squamous cell carcinoma antigen (SCCA) is an antigen that is extracted from squamous cell carcinoma cells, demonstrates high concentrations in the blood in squamous cell carcinoma of the cervical region, lungs, esophagus and skin, and is frequently used to diagnose squamous cell carcinoma (Kato, H., et al., Cancer, 40:1621-1628 (1977): NPL6; Mino, N. et al., Cancer, 62:730-734 (1988): NPL7). In particular, since SCCA levels in the blood exhibit a favorable correlation with such parameters as the stage of progression of squamous cell carcinoma, degree of malignancy or tumor size, it is a particularly effective cancer marker not only for early detection, but also for evaluating the efficacy of cancer therapy and diagnosing the risk of relapse.

SCCA is also known to demonstrate increased expression in the upper layer of psoriatic epithelium (Takeda, A., et al., J. Invest. Dermatol. (2002), 118(1), 147-154: NPL8). Psoriasis is a type of skin disease presenting with chronic, recurring inflammatory parakeratosis characterized by abnormal proliferation and division of epidermal cells and inflammatory cell infiltration. Psoriasis is thought to occur due to various environmental factors in combination with genetic factors (Hopso-Havu, et al., British Journal of Dermatology (1983), 109, 77-85: NPL9).

Mitsuishi, et al., Clin. Exp., Allergy (2005), 35:1327-1333 (NPL10) discloses that serum SCCA levels correlate with the severity of atopic dermatitis. However, there is not always a correlation between serum protein and serum protein present in corneocytes. Epidermal corneocytes proliferate in the basal layer, transfer to the upper layer, and differentiate into the corneal layer, and this corneal layer ultimately exfoliates from the skin. However, the corneal layer is regenerated through a phenomenon referred to as "enucleation" which is a characteristic state where the nuclei of corneocytes are eliminated as a result of normal cornification of the skin. During the course of corneocytes differentiating and losing their nuclei, some parts of endogenous proteins are degraded and are either lost or demonstrate a considerable reduction in the amount thereof due to the release of various endogenous proteases. Consequently, the environment to which proteins present in the corneal layer are exposed to is completely different from that in the bloodstream, and the amounts thereof exhibit behavior that is completely different from that in the blood. In contrast to serum TARC level serving as an indicator of the severity of atopic dermatitis, the reason why corneal layer TARC does not serve as an indicator thereof may possibly lie herein. Accordingly, even if there were findings indicating that serum SCCA correlates with the severity of atopic dermatitis, such findings do not suggest a correlation between SCCA in the corneal layer and the severity of atopic dermatitis. In particular, atopic dermatitis is a disease in which multiple eruptions are present that have different severities, and this is also clear from the fact that the findings of physicians were made on the basis of a plurality of parameters.

International Publication No. WO 2006/098523 (PTL 1) discloses that the SCCA of corneocytes (to also be referred to as corneal layer SCCA) serves as an indicator for predicting parakeratosis caused by atopic dermatitis. This publication indicates a finding that corneal layer SCCA can be used as an indicator to predict the risk of a subject not yet suffering from atopic dermatitis becoming afflicted with atopic dermatitis sometime in the future. Accordingly, it is clear that corneal layer SCCA is involved in the onset of atopic dermatitis. However, even if corneal layer SCCA is a causative factor of the onset of atopic dermatitis, it does not necessarily mean that corneal layer SCCA can also be used as an indicator of severity after the onset of disease. This is because, even if corneal layer SCCA was a causative factor of atopic dermatitis, there is the possibility that corneal layer SCCA is not involved with the progress of symptoms while instead the progress of symptoms after the onset may be governed by a different factor. In other words, a causative factor of a disease cannot always be used as a prognosis marker for that disease. In actuality, there are many examples of substances used as diagnostic markers for a disease being unable to be used as indicators of disease severity. For example, although the tumor marker, α-fetoprotein (AFP), is a diagnostic marker, the levels thereof do not coincide with the size of the tumor and the degree of increase in the levels thereof cannot be used to predict prognosis.

As stated at the outset, atopic dermatitis is a disease that undergoes repeated exacerbation and remission for which it is difficult to achieve a complete cure, and it is important to control symptoms during the course of treatment. Therefore, there is a desire among both physicians and patients when selecting a treatment strategy for a marker that is non-invasive and can serve as an indicator of local severity and can therefore serve as an objective indicator when explaining the treatment strategy to a patient. However, such a marker does not exist.

### [PRIOR ART DOCUMENT]

### [PATENT LITERATURE]

[PTL 1] WO 2006/098523

### [NON PATENT LITERATURE]

[NPL 1] The Japanese journal of dermatology, 119(8), 151-1534, 2009
[NPL 2] Japanese Guideline for the Diagnosis and Treatment of Allergic diseases 2013, Chapter 6, Atopic dermatitis
[NPL 3] Fiset PO, Leung DY. JACI; 118:287-290, 2006
[NPL 4] Sugawara et al., Allergy (2002) 57:180-181
[NPL 5] Morita et al., Allergy (2010)65: 1166-1172
[NPL 6] H.Kato et al.Cancer 40:1621-1628 (1977)
[NPL 7] N.Mino et al.Cancer 62:730-734 (1988)
[NPL 8] Takeda A.et al., J.Invest.Dermatol. (2002)118(1),147-154;
[NPL 9] Hopso-Havu et al.British Journal of Dermatology (1983)109,77-85
[NPL 10] Mitsuishi et al., Clin. Exp., Allergy (2005) 35;1327-1333

### SUMMARY

### [TECHNICAL PROBLEM]

An object of the present invention is to provide an objective and simple indicator of complete recovery and diagnosis of local and systemic severity of atopic dermatitis that does not rely on the skill of a physician, and particularly an experienced physician.

### [SOLUTION TO PROBLEM]

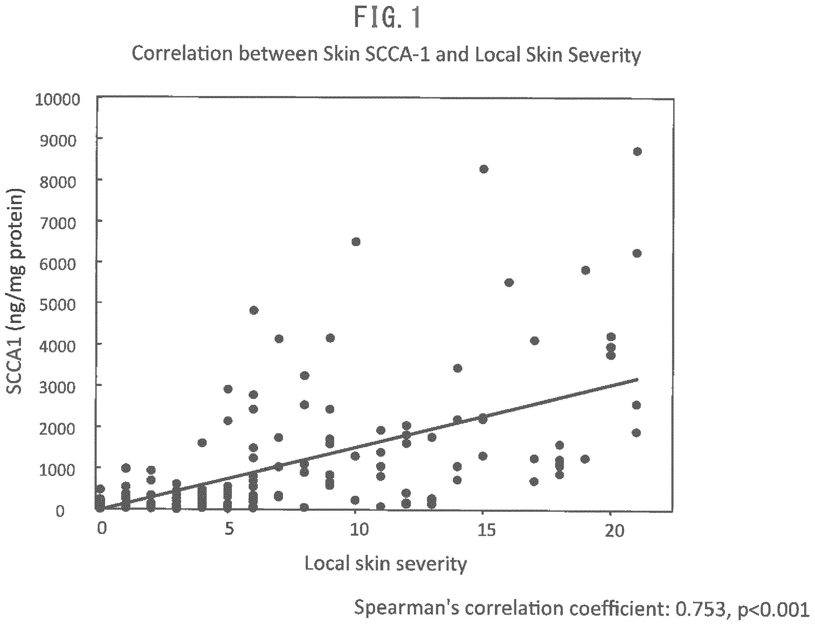

The inventors of the present invention investigated SCCA-1 expression levels in the corneocytes of subjects suffering from atopic dermatitis who were also undergoing treatment of that disease, together with visual skin findings based on a visual diagnosis of typical parameters considered to serve as indicators of atopic dermatitis by a physician. As a result, it was revealed that there was a high correlation between corneal layer SCCA-1 levels and severity of the disease as determined by the skin findings (Fig. 1). In other words, it was revealed that as the SCCA-1 levels increased, the skin findings were more serious, from remission to mild, moderate and severe. In addition, there are many cases in which subjects determine on their own that they have apparently made a complete recovery based on their own even though symptoms are still present according to the findings made by a physician. Under such circumstances, the inventors of the present invention found that the findings made by physicians generally coincided with a significant SCCA-1 level expression. In other words, the present invention offers the advantage of serving as an objective decision-making tool for enabling patients themselves to evaluate their own severity, and as a result thereof, makes it possible to prevent relapse or recurrence of disease caused by a failure to continue treatment stemming from the determination of having made a complete recovery according to the patient's own findings despite the disease requiring further treatment and continued monitoring by a physician. As a result, there is also the advantage of being able to prevent relapse or recurrence of disease not by leaving the disease per se completely untreated, if SCCA-1 levels are higher than those of healthy subjects, but by performing skin care through the use of moisturizers and protective agents, for example, when a patient has been determined to have entered remission. Moreover, if expression of SCCA-1 is high, exacerbation of symptoms can be prevented since a determination can be made as to whether or not there is the need for treatment based on topical steroid preparations or non-steroid topical preparations having anti-inflammatory effects.

As described above, local severity can be determined in subjects currently undergoing treatment for atopic dermatitis, subjects who have undergone treatment in the past, subjects who are currently in remission, and subjects who appear healthy but have the potential for the onset of atopic dermatitis in the future, by using SCCA-1 present in corneocytes as an indicator.

The present application provides the following inventions.
(1) A diagnosis assisting method for determining a treatment strategy of atopic dermatitis in a subject undergoing the treatment thereof,
   wherein the method comprises:
   measuring the SCCA-1 expression level in corneocytes of the subject; and
   i) if the SCCA-1 expression level of the subject is not statistically significantly higher than the SCCA-1 expression level of those who do not have atopic dermatitis, discontinuing the treatment and determining that there is no need of continuing the treatment.
(2) A diagnosis assisting method for determining a treatment strategy of atopic dermatitis in a subject undergoing the treatment thereof,
   wherein the method comprises:
   measuring the SCCA-1 expression level in corneocytes of the subject; and
   ii) classifying the severity of atopic dermatitis of the subject by using the SCCA-1 expression level as an indicator, and determining to apply a treatment method for atopic dermatitis corresponding to respective class to the subject.
(3) A diagnosis assisting method for determining the necessity of the treatment of atopic dermatitis in a subject in need of such a determination or a subject undergoing the treatment thereof, or for determining a treatment strategy of atopic dermatitis if it is determined that the treatment thereof is necessary,
   wherein the method comprises:
   measuring the SCCA-1 expression level in corneocytes of the subject; and
   i) if the SCCA-1 expression level of the subject is not statistically significantly higher than the SCCA-1 expression level of those who do not have atopic dermatitis, determining that there is no need of conducting the treatment or continuing the treatment if the subject is undergoing the treatment; or
      if the SCCA-1 expression level of the subject is statistically significantly higher than the SCCA-1 expression level of those who do not have atopic dermatitis, determining that there is a need of conducting the treatment or continuing the treatment if the subject is undergoing the treatment, and furthermore
   ii) classifying the severity of atopic dermatitis of the subject by using the SCCA-1 expression level of the subject as an indicator, and determining to apply a treatment method for atopic dermatitis corresponding to respective class to the subject.
(4) The diagnosis assisting method according to any one of (1) to (3), wherein said determination of the treatment strategy is repeatedly performed over a long term.
(5) The diagnosis assisting method according to any one of (1) to (4), wherein said severity of atopic dermatitis is regarding one or more items selected from the group consisting of erythema, oozing, papulation, excoriation, lichenification, xerosis/dryness and itch.
(6) The diagnosis assisting method according to any one of (1) to (5), wherein said skin corneocytes are collected in a non-invasive manner by tape stripping.
(7) The diagnosis assisting method according to any one of (1) to (6), wherein said measurement of the SCCA-1 expression level is performed by immunoassay using an SCCA-1 specific antibody.
(8) The diagnosis assisting method according to (7), wherein said immunoassay is selected from the group consisting of enzyme immunoassay (including ELISA, EIA), fluorescent immunoassay, radioimmunoassay (RIA method), luminescent immunoassay, surface plasmon resonance (SPR), quartz crystal microbalance (QCM), immune turbidimetry, latex agglutination immunoassay, latex turbidimetry, particle agglutination reaction method, gold colloid method, capillary electrophoresis, graphene biosensor, amperometric biosensor, laser spectrometry, and surface enrichment immunoassay.
(9) The diagnosis assisting method according to (7) or (8), wherein said collection of skin corneocytes and said measurement of the SCCA-1 expression level is conducted at home or medical checkup.
(10) The diagnosis assisting method according to any one of (1) to (9), wherein said collection by tape stripping is performed on three or more sites of the skin of the subject.

### [ADVANTAGEOUS EFFECTS OF INVENTION]

The SCCA-1 level in the corneal layer makes it possible to non-invasively determine local severity of atopic dermatitis. Moreover, the SCCA-1 level in the corneal layer also serves as an objective indicator for determining an effective treatment strategy for subjects with atopic dermatitis as well as subjects currently undergoing treatment thereof. Thus, a persuasive and objective indicator for determining the proper treatment strategy according to severity of the disease or for determining a treatment strategy that enables remission to be sustained for as long as possible is provided that is also required for explaining future treatment strategies to patients. More specifically, it has become possible to determine a treatment strategy such as whether or not topical steroid preparations are to be continued or whether or not topical preparations other than tacrolimus or other steroids having anti-inflammatory effects are to be continued. In addition, if it were possible to use SCCA-1 level in the corneal layer as an indicator, the development of a simple assay kit for measuring SCCA-1 levels in the corneal layer would make it possible for a patient him/herself to objectively determine whether his own condition requires treatment at home without having to visit a hospital and without seeking the diagnosis of a physician. Alternatively, a patient's condition could be determined at an early stage such as during a routine medical examination. As a result, it would be possible to prevent relapse or recurrence of disease caused by neglecting to continue treatment as a result of the patient determining treatment to not be required based on the patient's own findings despite the disease still requiring further treatment. Alternatively, it would be possible to respond quickly by determining the required treatment method. Differences in treatment attributable to differences in the skill levels of physicians would also be reduced. Moreover, since the method of the present invention uses corneal layer SCC1-a as an indicator, the method of the present invention does not require collection of blood and is non-invasive. When considering that infants and young children account for 89% of the incidence of atopic dermatitis, the method of the present invention is extremely effective in terms of eliminating patient burden attributable to pain associated with blood collection while also eliminating technical difficulty in the health care setting. In addition, as a result of collecting corneal layer samples from multiple locations, such as three locations or more, for a single subject and using the average SCCA-1 level thereof as an indicator, a simple and reliable method for classifying the systemic severity of atopic dermatitis can be provided to take the place of SCORAD or EASI, which are frequently used to classify the severity of atopic dermatitis.

### BRIEF DESCRIPTION OF DRAWINGS

FIG. 1 indicates that corneal layer SCCA-1 levels demonstrate a high correlation with severity of atopic dermatitis (local skin severity).
FIG. 2 is a bar graph indicating that corneal layer SCCA-1 levels demonstrate a high correlation with the severity of atopic dermatitis (local skin severity).
FIG. 3-1 depicts bar graphs respectively indicating that corneal layer SCCA-1 levels demonstrate a high correlation with the severity of various skin findings associated with atopic dermatitis.
FIG. 3-2 depicts bar graphs respectively indicating that corneal layer SCCA-1 levels demonstrate a high correlation with the severity of various skin findings associated with atopic dermatitis.
FIG. 4 is a graph indicating that corneal layer TARC levels demonstrate a low correlation with the severity of atopic dermatitis.
FIG. 5 depicts graphs indicating dissociation between local skin findings and patients' own findings of the severity of atopic dermatitis.
FIG. 6-1 depicts graphs indicating monitored results of the progress of treatment of patients having atopic dermatitis obtained by using SCCA-1 and skin findings determined by a physician as indicators.
FIG. 6-2 depicts graphs indicating monitored results of the progress of treatment of patients having atopic dermatitis obtained by using SCCA-1 and skin findings determined by a physician as indicators.
FIG. 6-3 depicts graphs indicating monitored results of the progress of treatment of patients having atopic dermatitis obtained by using SCCA-1 and skin findings determined by a physician as indicators.
FIG. 7 depicts graphs indicating monitored results of the severity of atopic dermatitis obtained by using SCCA-1 and skin findings determined by a physician in subjects having elevated SCCA-1 levels as indicators.
FIG. 8 depicts graphs indicating monitored results of the severity of atopic dermatitis obtained by using SCCA-1 and skin findings determined by a physician in subjects having slightly higher SCCA-1 levels than healthy subjects as indicators.

### DESCRIPTION OF EMBODIMENTS

### SCCA-1

SCCA is encoded by two genes consisting of an SCCA-1 gene and SCCA-2 gene arranged in tandem on chromosome 18q21.3. The proteins SCCA-1 and SCCA-2 encoded thereby are both proteins having a molecular weight of about 45,000, exhibit high homology, and the homology thereof is 95% at the nucleic acid level. These SCCA belong to the family of ovalbumin-serine protease inhibitors (ov-serpins). Ov-serpins have the most unique characteristics among members of the serpin superfamily. Although serpins are typically excreted and act outside cells, ov-serpins mainly act as protein inhibitors within cells.

Although SCCA-1 is a papain-like cysteine protease inhibitor, SCCA-2 is a chymotrypsin-like serine protease inhibitor, and since their amino acid sequences at the reaction site differ despite having a high degree of homology, demonstrate different properties (Schick, et al., J. Biol. Chem. (1997), 27213, 1849-1855). The inventors of the present invention found that SCCA-1 in particular can serve as an indicator of the severity of atopic dermatitis by a non-invasive means.

### Atopic Dermatitis

The following definitions and explanations of terms are as a general rule based on the Guidelines for the Treatment of Atopic Dermatitis of the Japanese Dermatological Association.

### <Atopic Dermatitis>

Atopic dermatitis is a disease that undergoes repeated exacerbation and remission and has eczema with pruritus as the primary etiology thereof and many patients have a predisposition to atopic dermatitis (have an eruption that differs in severity even within the same person).

### <Diagnostic Criteria of Atopic Dermatitis>

In general, persons satisfying the three basic parameters of pruritus, distribution of characteristic eruption and chronic/repetitive progression (for 2 months of more in infants and small children and 6 months or more in others) are diagnosed with atopic dermatitis.

### <Severity of Eruption of Atopic Dermatitis and Selection of Topical Preparation>

According to the Guidelines for the Treatment of Atopic Dermatitis of the Japanese Dermatological Association, the severity of eruption associated with atopic dermatitis can be classified into classes consisting of remission, minor, weak, medium and severe. The following provides an explanation of each class of severity along with an indication of the preferred treatment method.
1) Severe
   * Eruption severity: Mainly consists of advanced erythema accompanied by swelling, edema, oozing or lichenification, numerous papules, advanced xerosis, crusting, small blisters, erosion, multiple excoriation and itchy nodules.
   * Selection of topical preparation: A very strong or strong class topical steroid preparation that demonstrates required and adequate effects is the first choice of treatment. The strongest class of topical steroid preparation may also be used by only at the affected site in the case adequate effects are unable to be obtained for itchy nodules with very strong topical steroid preparations.
2) Medium
   * Eruption severity: Consists mainly of moderate erythema, xerosis, small number of papules and excoriations.
   * Selection of topical preparation: A strong or medium class topical steroid preparation is the first choice of treatment.
3) Weak
   * Eruption severity: Consists mainly of dryness, milk erythema and xerosis.
   * Selection of topical preparation: A medium or weaker class of topical steroid preparation is the first choice of treatment.
4) Minor
   * Eruption severity: Consists mainly of dryness symptoms lacking inflammatory symptoms.
   * Selection of topical preparation: A steroid-free topical preparation is selected.
5) Remission: Refers to a state in which symptoms attributable to the illness have improved, disappeared or are clinically controlled regardless of whether permanent or temporary. Namely, remission refers to a state that does present a problem clinically without necessarily being completely healed. This state also includes the possibility of exacerbation at some point in the future (which differs from being completely healed or a complete cure).
   * Selection of topical preparation: Steroid-free topical preparation or moisturizer and the like are selected.

The aforementioned classes of severity are naturally only examples, and eruption severity may also be classified as absence of symptoms, mild, moderate or severe. In the case of such classification, treatment strategies may be established, such as the application of steroid-free topical preparations to mild cases, the application of medium class topical steroid preparations to moderate cases, and the application of very strong or strong class topical steroid preparations to severe cases (Japanese Guidelines for the Diagnosis and Treatment of Allergic Diseases 2013, Chapter 6: Atopic Dermatitis (NPL2)).

The following indicates definitions of those terms used in the subsequent explanations.
* Eruption: A lesion appearing on the skin is generically referred to as an eruption or rash.
* Dermatitis: Inflammation occurring on the skin (accounting for 1/5 to 1/3 of all dermatitis out-patient examinations). The inflammatory reaction occurring on the skin appears in the form of eczema. Clinically presents with pruritus, redness, xerosis and papulation.
* Eczema: Same meaning as dermatitis.
* Erythema: Red patches of skin occurring due to vasodilation and congestion in dermal papilla and subpapillary layer.
* Papule: Refers to a localized protruding change (protrusion of the skin) having a diameter of 1 cm or less.
* Nodule: Nodule refers to a localized protruding change similar to a papule that has a diameter of 1 cm or more to about 2 to 3 cm. Nodules are deeper than papules and extend to the dermis or subcutaneous tissue.
* Lichen: Papule that have entered a permanent state.
* Oozing: State in which leukocytes and lymphocytes have entered the skin at the site of a lesion where dermatitis has occurred. May also refer to a state in which the focus gradually enters surrounding normal tissue.
* Crust, Crusting: Result of solidification of keratin, exudate, blood, purulent matter or necrotic tissue. Also known as a so-called "scab".
* Pruritus: Itchiness

Examples of the ranks of the strength of the pharmacological efficacy of topical steroid preparations are shown in Table 1 below. Furthermore, these ranks of the strength of the pharmacological efficacy of these preparations are merely intended to be exemplary.

**[Table 1]**

| Strongest Class |
|---|
| 0.05% clobetazol propionate (Dermovate ®) |
| 0.05% diflorasone diacetate (Diflal®, Diacort®) |

| Very Strong Class |
|---|
| 0.1% mometasone furoate hydrate (Fulmeta®) |
| 0.05% betamethasone butyrate propionate (Antebate ®) |
| 0.05% fluocinonide (Topsym ®) |
| 0.064% betamethasone dipropionate (Rinderon ®) |
| 0.05% difluprednate (Myser®) |
| 0.1% amcinonide (Visderm®) |
| 0.1% diflucortolone valerate (Texmeten ® ,Nerisona®) |
| 0.1% hydrocortisone butyrate propionate (Pandel ®) |

| Strong Class |
|---|
| 0.3% deprodone propionate (Ecler ®) |
| 0.1% dexamethasone propionate (Methaderm ®) |
| 0.12% dexamethasone valerate (Voalla ®, Zalucs®) |
| 0.1% halcinonide (Adcortin ®) |
| 0.12% betamethasone valerate (Betnevate ®, Rinderon®) |
| 0.025% beclometasone dipropionate (Propaderm ®) |
| 0.025% fluocinolone acetonide (Flucort ®) |

| Medium Class |
|---|
| 0.3% prednisolone valerate acetate (Lidomex ®) |
| 0.1% triamcinolone acetonide (Ledercort ®, Kenacort®) |
| 0.1% alclometasone dipropionate (Almeta ®) |
| 0.05% clobetasone butyrate (Kindavate ®) |
| 0.1% hydrocortisone butyrate (Locoid ®) |
| 0.1% dexamethasone (Glymesason ®, Oirazone®) |

| Weak Class |
|---|
| 0.5% prednisolone (Prednisolone ®) |

The absolute value of the expression level of corneal layer SCCA-1 would fluctuate depending on the measurement system used to measure corneal layer SCCA-1. Thus, in order to assign a subject to the proper severity corresponding to the expression SCCA-1 level, in the measurement system used to measure corneal layer SCCA-1, it is necessary to prepare a calibration curve that indicates the relationship between corneal layer SCCA-1 expression level and severity. In order to prepare a calibration curve, it is first preferable to preliminarily obtain skin visual findings through examination by a physician. Examples of parameters of the findings include the presence of drying symptoms, erythema, xerosis, papulation, excoriation, swelling, edema, crusting, small blisters, erosion or itchy nodules. Each finding is evaluated for severity based on evaluation criteria, for example, 0: absence, 1: mild, 2: moderate and 3: severe. Although the number of subjects used to prepare the calibration curve is preferably larger, for example, 50 or more subjects can be used, the number of subjects is not limited thereto. The scores for each of these parameters are then totaled, and a calibration curve is prepared by plotting from 0 points (i.e., the case where the scores for all parameters are zero) to the maximum number of points (i.e., the case where the scores for all parameters are 3) on the horizontal axis and plotting measured values for corneal layer SCCA-1 on the vertical axis. The horizontal axis can be assigned to suitably classified severity with respect to, for example, healthy (normal), remission, mild, moderate and severe, starting from the lowest score. Once the SCCA-1 value of a subject has been obtained, that value is applied to the calibration curve to determine to which level of severity the severity of the affected area of the subject applies.

By presenting the patient with an objective indicator of severity, the patient can be persuaded that a treatment strategy using steroid preparations is necessary even if the patient has an incorrect awareness of steroid preparations, thereby making it possible to apply an effective treatment strategy that matches the severity of that patient. Since atopic dermatitis is a disease for which treatment extends over a long period of time, it is necessary to create trusting relationship between the physician and patient to maintain motivation of the patient to receive treatment, the present indicator is thought to be useful.

Once this type of calibration curve has been prepared, the corresponding severity can be determined and a treatment strategy suitable for that severity can be established simply by measuring the expression level of corneal layer SCCA-1 of the subject using this measurement system. Treatment strategies suitable for each severity can be determined in accordance with, for example, guidelines for the treatment of atopic dermatitis. For example, a topical steroid preparation of the strongest, very strong or strong class can be used as the first choice of treatment in line with these guidelines for an affected area of a subject for which severity has been determined to be severe based on the expression level of corneal layer SCCA-1. Steroid-free topical preparations or moisturizers and the like can be selected in line with these guidelines for an affected area of a subject for which severity has been determined to be mild based on the expression level of corneal layer SCCA-1.

Atopic dermatitis also has a so-called state of remission in which it is determined based on external visual findings that the disease does not exist. In the case of "remission", it is proven that for example even if eczema temporarily improves through the use of an anti-inflammatory drug, there are cases where perivascular lymphocytes which are low-level in terms of histology cause inflammatory cell infiltration, swelling of endothelial cells and mild lesions accompanying basement membrane thickening (Fiset, P.O., Leung, D.Y., JACI, 188:287-290,2006) (NPL2). Since it is extremely difficult to determine skin conditions during remission based on external visual findings, in conventional treatments so-called "reactive therapy" have been employed in which medication is switched over to skin care using moisturizers and the like during remission, and once visibly recognizable inflammation has returned, medication is switched over to steroids and tacrolimus.

Since conventional methods do not have an objective indicator to evaluate whether or not atopic dermatitis requires treatment, clinical findings from experienced physicians were always sought. According to the present invention, the state of remission can be evaluated in greater detail thereby making it extremely advantageous. In order to accomplish this, the corneal layer SCCA-1 levels of a plurality of healthy subjects not suffering from atopic dermatitis are first measured. Although there are no particular limitations on the number of healthy subjects measured, the number of subjects is preferably larger, for example, the number may be 10 or more, preferably 20 or more and most preferably 50 or more, although not limited thereto. By so doing, the corneal layer SCCA-1 levels of healthy subjects can be determined. If the corneal layer SCCA-1 level of a subject is statistically significantly higher than the above determined value, it is understood that atopic dermatitis is in a state significantly susceptible to recurrence.

The present invention can be applied to so-called proactive therapy (preventive intermittent application) in which intermittent application of a mild anti-inflammatory agent and skin care with moisturizers and protective agents is performed periodically during remission, and once the stage has been reached for which treatment is no longer necessary, skin care is switched over to the usage of only moisturizers and protective agents.

It is preferable to measure the corneal layer SCCA-1 expression level by using a kit for measuring corneal layer SCCA-1 with a preliminarily prepared calibration curve, for example by using a commercial assay kit. As a result, preparation of a calibration curve can be omitted and any level of severity can be determined simply by measuring the expression level of corneal layer SCCA-1 of a subject in this measurement system, thereby making it possible to determine the treatment strategy suitable for that severity.

Measurement of the expression of SCCA-1 according to the present invention can be carried out qualitatively or quantitatively in accordance with any arbitrary method that enables measurement of SCCA-1. More specifically, various methods can be used, for example, immunoassays using antibody specific to SCCA-1 such as ELISA using enzyme labeling, RIA using radioactive labeling, immunonephelometry, western blotting, latex agglutination, hemagglutination, immunochromatography, plasmon resonance, surface acoustic wave (SAW) device or electronic device. Examples of immunoassays include competitive methods and sandwich methods. In addition, the expression SCCA-1 level can also be determined by measuring the amount of SCCA-1 expressed in cells by a gene encoding the same. In this case, the expression of SCCA-1 is preferably determined by measuring the amount of mRNA encoding intracellular SCCA-1. Extraction of mRNA and other quantitative or qualitative methods for determining the amount thereof can be carried out in accordance with known methods, such as a PCR method, a 3SR method, an NASBA method, and a TMA method. In addition, expression of SCCA-1 can be qualitatively determined by in situ hybridization or measurement of the biological activity thereof.

Although collection of a sample of the skin corneal layer serving as a specimen can be carried out by any arbitrary method, tape stripping is preferable from the viewpoint of simplicity. Tape stripping refers to attaching a piece of adhesive tape to the skin surface layer and peeling off the tape to enable the skin surface layer to adhere to the peeled adhesive tape and collect a sample of the corneal layer. Use of the tape stripping method makes it possible to measure expression of SCCA-1 simply by sampling the corneal layer with a single piece of tape, thereby making it possible to determine the state of atopic dermatitis using SCCA-1 as an indicator. A preferable method for tape stripping consists of first removing any sebaceous matter and dirt from the surface layer of the skin, gently placing a piece of adhesive tape cut to a suitable size (such as 2 cm × 6 cm) on the skin surface, uniformly pressing the tape onto the skin by applying even force over the entire piece of tape, and subsequently peeling off the adhesive tape with uniform force. Examples of methods used to remove sebaceous matter and dirt include wiping the skin with tissue paper, washing the skin with soap and cleaning the skin with water or ethanol. Commercially available cellophane tape may be used for the adhesive tape, and examples of tape that can be used include Scotch Super Strength Mailing Tape (3M Corp.) and Cellophane Tape (Cellotape®, Nichiban Co., Ltd.). SCCA-1 present in the skin corneal layer sample adhered to the adhesive tape can be isolated and extracted from the tape by immersing the piece of tape in a suitable extraction liquid such as Tris buffer (pH 8.0) (0.1 M Tris-HCl, 0.14 M NaCl, 0.1% Tween-20) and extracting the corneal layer.

In a preferable aspect of the present invention, SCCA-1 is measured using an immunoassay method such as ELISA. The antibody specific for SCCA-1 used in ELISA may be monoclonal antibody or polyclonal antibody. Methods for producing monoclonal antibody and polyclonal antibody are known among persons with ordinary skill in the art, and are described in, for example, Lunstrum, et al., J. Biol. Chem., 1986, 261: 9042-9048 and Hurle, et al., J. Cell Science, 1994, 107: 2623-2634.

In the method according to the present invention, a sandwich immunoassay is particularly preferable. A sandwich immunoassay can be carried out, for example, as indicated below.

One of two types of antibody specific for SCCA-1 is immobilized on a carrier as primary antibody. The carrier is preferably a solid carrier, any arbitrary solid carrier routinely used in immunoassays may be used for the solid carrier, and examples thereof include styrene, polystyrene or other polymer carriers formed to an arbitrary size and shape, and reaction vessels formed with these materials such as the inner walls of the wells of an ELISA plate.

Immobilization of the aforementioned primary antibody to the carrier can be carried out in accordance with an ordinary method, and for example, the aforementioned primary antibody can be immobilized by dissolving in a buffer solution such as phosphate-buffered saline (PBS) or borate buffer and adsorbing to the carrier. In addition, an antibody that binds to the aforementioned primary antibody or another protein such as protein c may be preliminarily immobilized on the carrier followed by contacting with the aforementioned primary antibody. Moreover, in order to prevent non-specific binding, a suitable blocking agent, such as PBS-BSA or a commercially available blocking agent such as Block Ace (Dainippon Pharmaceutical Co.), is added to the carrier having the primary antibody immobilized thereon followed by incubating at about 4°C to 40°C and preferably 20°C to 37°C for 5 minutes to several days, preferably for 10 minutes to 24 hours and more preferably for 10 minutes to 3 hours for the purpose of blocking non-specific binding.

The other of the aforementioned two types of antibody specific for SCCA-1 is labeled for use as secondary antibody. Examples of labeling include enzyme labeling, radioactive isotope labeling and fluorescent labeling. In the case of enzyme labeling, the antibody can be labeled by binding the enzyme directly to the antibody or the antibody can be labeled with enzyme indirectly through mutually reactive proteins in the manner of avidin and biotin. Binding of enzyme to antibody can be carried out using, for example, a commercially available thiol group introduction reagent by introducing a thiol group into each enzyme and antibody to be labeled followed by binding the two moieties with disulfide (S-S) linkages. Examples of enzymes include horseradish peroxidase, alkaline phosphatase and β-D-galactosidase. Enzyme extraction can be carried out using a substrate specific for the enzyme. For example, in the case of using horseradish peroxidase, a substrate such as 3,3',5,5'-tetramethylbenzidine (TMB) or 2,2'-azino-di-[3-ethylbenzthiazoline sulfonate (ABTS) can be used.

This immunoassay causes SCCA-1 present in a sample to bind to the aforementioned primary antibody immobilized on a carrier and the aforementioned labeled secondary antibody to bind to this SCCA-1 molecule by mixing the carrier having the primary antibody immobilized thereon, the labeled secondary antibody and a test sample.

In this manner, the labeled antibody is immobilized on the carrier through the primary antibody immobilized on the carrier and SCCA-1 derived from the sample in an amount that reflects the amount of SCCA-1 in the sample. Incubation is carried out in a suitable buffer solution such as PBS at about 4°C to 40°C and preferably 20°C to 37°C for 5 minutes to several days, preferably 10 minutes to 24 hours and more preferably 10 minutes to 3 hours.

Next, a procedure is carried out for separating the labeled antibody from the aforementioned carrier. In the case the carrier is a solid carrier, this separation procedure can be carried out easily by solid-liquid separation. In the case of using a certain known amount of labeled secondary antibody, the label bound to the carrier, unbound label or both can be measured. On the other hand, in the case of having used an arbitrary labeled antibody, the label bound to the carrier is detected and measured. In order to detect the label bound to the carrier, the carrier is preferably washed with a washing solution such as a buffer such as PBS-Tween 20 containing a suitable surfactant to remove unbound labeled antibody followed by detection thereof. Detection can be carried out in accordance with ordinary methods depending on the type of label.

The following provides a more detailed explanation of the present invention by indicating specific examples thereof. Furthermore, the present invention is not limited thereby.

### EXAMPLES

### 1. Evaluated and Observed Parameters

### (1) Local Skin Findings obtained by a Physician

Parameters consisting of (A) erythema, (B) oozing, (C) papulation, (D) excoriation, (E) lichenification, (F) xerosis/dryness and (G) itch were observed at each site, and the severities thereof were filled in case cards.

The evaluation criteria consisted of 0 (absence), 1 (mild), 2 (moderate) and 3 (severe). Healthy sites, eruption sites and eruption-free sites where the corneal layer was sampled were described in a full body diagram.

### (2) Collection and Measurement of Corneal Layer Tape

The corneal layer of the test site was collected using Cellotape® (2 cm × 6 cm). The resulting corneal layer sample was immersed in a protein extraction liquid (0.1 M Tris-HCl (pH 8.0), 0.14 M NaCl, 0.1% Tween-20) and extracted with a Bioruptor. The extract was then centrifuged and the resulting supernatant was used for ELISA.

### (3) Immunological SCCA-1 Assay Method

Recombinant SCCA-1 was obtained by inserting SCCA-1 DNA into a pQE30 vector. After purifying the recombinant with Ni-Agarose, the recombinant was further purified by Mono Q Chromatography and SCCA-1 was measured using ELISA assay for the standard. Namely, rabbit anti-SCCA-1 polyclonal antibody (Shiseido Japan) was used as primary antibody by immobilizing on a 96-well plate. After blocking with ImmunoBlock (DS Pharma Biomedical, Osaka, Japan), 100 µl aliquots of corneal layer extract were added to each well followed by allowing to react by incubating the plate at 37°C for 1 hour. The reaction was allowed to further react for 1 hour at 37°C after adding secondary antibody in the form of monoclonal anti-SCCA-1 antibody (Santa Cruz). Subsequently, horseradish peroxidase-conjugated anti-mouse F(ab')2 (GE Healthcare) was added followed by developing color with the TMB Peroxidase EIA Substrate Kit (Bio-Rad). The reaction was stopped with 1 M H₂SO₄ and measurement was carried out at 450 nm.

### (4) Preparation of Calibration Curve

A test was conducted using 52 healthy subjects and 189 atopic dermatitis patients as subjects. The healthy subjects were diagnosed as not having an allergic disease (including atopic dermatitis) by a physician. Qualifying conditions of the atopic dermatitis patients consisted of 1) currently diagnosed, or having been previously diagnosed in the past, with atopic dermatitis by a physician, 2) are able to not use the aforementioned drugs at the test site for one week or more from before the start of the test, and 3) age 20 or older.

Atopic dermatitis exclusion criteria consisted of 1) undergone medicinal treatment, including steroids or immunosuppressants, for one month or more from before the start of the test, 2) undergone treatment with a topical steroid preparation, tacrolimus hydrate ointment, non-steroidal anti-inflammatory drug or other topical preparation (including over-the-counter drugs) at the test site for one week or more from before the start of the test, or unable to not use the aforementioned drugs for one week or more from before the start of the study, although application of moisturizer is permitted, 3) presence of skin disease other than atopic dermatitis at the test site, 4) currently pregnant or lactating, and 5) judged to be unsuitable for participation in the test by the test director, participating physician or cooperating physician.

SCCA-1 levels were measured for the healthy subjects and atopic dermatitis patients. Skin findings at the test site were scored for the atopic dermatitis patients for (A) erythema, (B) oozing, (C) papulation, (D) excoriation, (E) lichenification, (F) xerosis/dryness and (G) itch by evaluating in line with the evaluation criteria described in the title column entitled "Local Skin Findings", and severity at the test site was determined from the total scores of each parameter of the skin findings. The total score of each subject was plotted on the horizontal axis and the corresponding SCCA-1 level was plotted on the vertical axis to obtain the graph shown in Fig. 1. Fig. 1 indicates the correlation between skin severity and corneal layer SCCA-1 levels in atopic dermatitis, Spearman's correlation coefficient demonstrated a high value of 0.753 (p<0.001), and a significantly strong correlation was indicated between skin findings determined by a physician and skin SCCA-1 levels.

Moreover, the total scores of the skin finding parameters of the atopic dermatitis patients were ranked into three classes consisting of 0.1 to 7 points, 8 to 14 points and 15 to 21 points, and in addition to calculating the average SCCA-1 levels for the healthy subjects, average SCCA-1 levels were also calculated for each class. Furthermore, patients having a total score of 0 were patients who, although suffering from atopic dermatitis, were not observed to exhibit any of the finding parameters at the test site. As a result, the average SCCA-1 level was 19.1 ng/mg among healthy subjects and 86.3 ng/mg among patients having a total finding parameter score of 0 (remission), 415.7 ng/mg among patients having a total finding parameter score of 1 to 7 points (mild), 1380.5 ng/mg among patients having a total finding parameter score of 8 to 14 points (moderate), and 1930.9 ng/mg among patients having a total finding parameter score of 15 to 21 points (severe).

Fig. 2 indicates the average values of corneal layer SCCA-1 levels for each class with a bar graph. Corneal layer SCCA-1 levels can be understood to become higher as severity as determined by a physician according to skin findings becomes higher. Thus, the degree of severity of a patient can be determined objectively and can be made to be useful for determining a treatment strategy by using SCCA-1 as an indicator. In addition, it is interesting to note that the average value of SCCA-1 levels of the patient group for which the total value of skin findings as determined by a physician was zero were statistically significantly higher than the average value of the healthy subjects. In other words, there are cases in which local skin condition can be evaluated by using SCCA-1 as an indicator even at locations that are apparently free of symptoms. Thus, the use of SCCA-1 as an indicator makes it possible to determine local skin condition for which determination thereof has conventionally been difficult even by a physician. As a result, patients can be prevented from discontinuing treatment and symptoms can be controlled.

Figs. 3-1 to 3-2 depict bar graphs obtained by calculating the average values of SCCA-1 levels according to score for each of the aforementioned skin finding parameters (A) to (G), plotting the score for each parameter on the horizontal axis and plotting average SCCA-1 levels on the vertical axis. SCCA-1 levels and the severity of each finding parameter exhibited an extremely high correlation. In the cases of papulation and xerosis/dryness in particular, a significant difference (p<0.001) was observed between the patient group having a finding score of 0 and the healthy subjects. Accordingly, the use of SCCA-1 as an indicator enables an objective determination of the degree of local skin severity of a patient for all typical finding parameters associated with symptoms of atopic dermatitis, and can be made to be useful for determining a treatment strategy suitable for each finding parameter.

### 2. Measurement of TARC

### Preparation of Calibration Curve

The correlation between corneal layer TARC levels and skin findings was investigated in the same manner as SCCA-1. Measurement of corneal layer TARC was carried out by applying a resulting corneal layer extract to ELISA. The Quantikine ELISA Human CCL17/TARC kit (R&D Systems) was used to carry out ELISA.

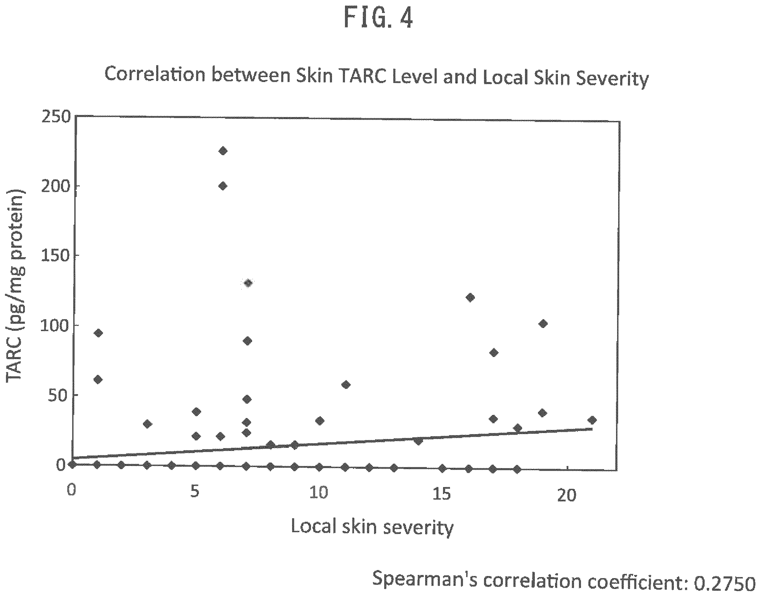

The results are shown in Fig. 4. Spearman's correlation coefficient between local skin severity and corneal layer TARC1 levels in atopic dermatitis was low at 0.2750, and corneal layer TARC was indicated as not being able to be used as an indicator of severity of atopic dermatitis.

### 3. Local Skin Findings as Determined by Physician and Self-Determined Findings

There are cases in which determination of the severity of atopic dermatitis is difficult even by an experienced physician, and determination of severity is extremely difficult by an ordinary person without any medical experience. In the present example, an investigation was made of the dissociation between skin findings made by an experienced physician and the patient him/herself during examination of atopic dermatitis.

A physician and the patient him/herself observed each of the parameters of (A) erythema, (B) oozing, (C) papulation, (D) excoriation, (E) lichenification, (F) xerosis/dryness and (G) itch at each site for two subjects exhibiting comparatively mild severity of atopic dermatitis. Evaluation criteria consisted of 0: absence, 1: mild, 2: moderate and 3: severe. Healthy sites, eruption sites and non-eruption sites where samples of the corneal layer were collected were described in a full body diagram.

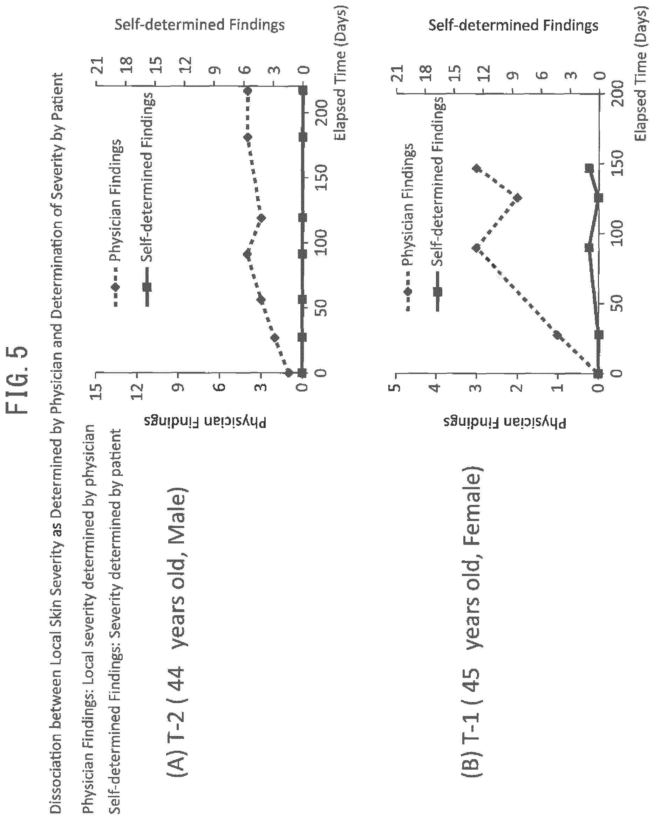

The results are shown in Fig. 5. Self-determined findings exhibited clearly lower values than the findings determined by a physician for both subjects. Thus, patients can be understood to tend to evaluate self-determined findings lower particularly in the case of mild severity of atopic dermatitis. Therefore, it can be understood that atopic dermatitis is a disease in which symptoms easily recur as a result of interrupting or discontinuing treatment even though the treatment is required. As mentioned above, corneal layer SCCA-1 can be collected using an extremely simple and non-invasive procedure in the form of tape stripping. Since measurement of SCCA-1 can be performed not only at a hospital or testing facility but also at home by developing an easy assay kit, SCCA-1 is an extremely superior indicator for evaluating the severity of atopic dermatitis and the efficacy of current treatment. Since infants and small children account for the majority of atopic dermatitis patients in particular, being able to determine the severity of the disease on one's own at home is extremely advantageous for patients who are unable to convey their symptoms on their own or for whom it is not easy to visit a hospital.

### 5. Monitoring of Treatment Progress

### Monitoring of Treatment Progress 1-1

### Patient TA04-1

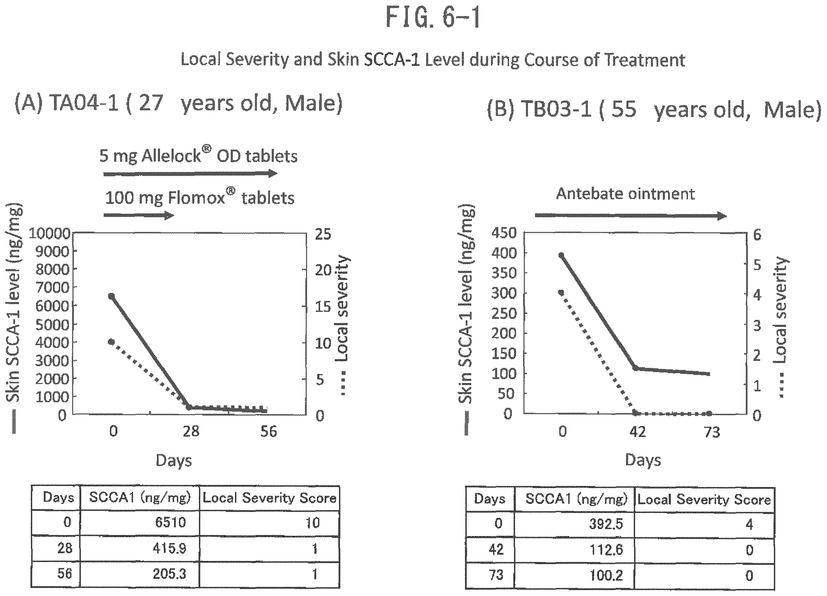

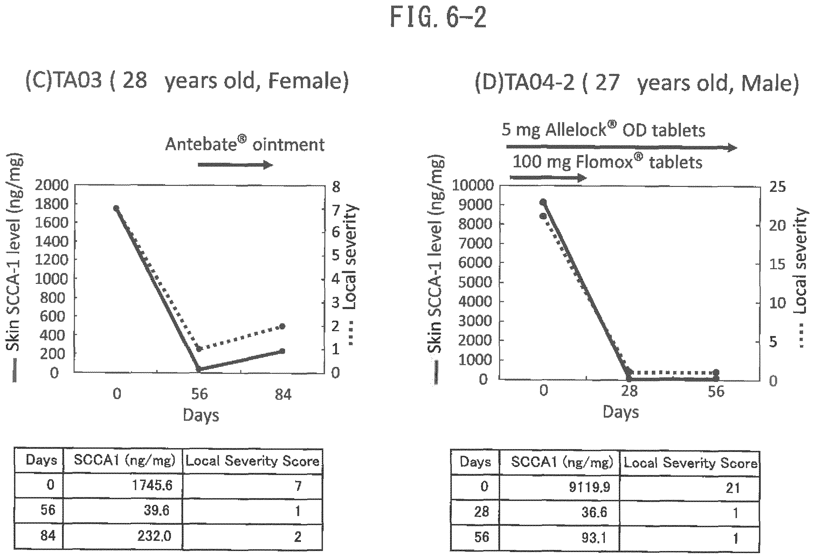

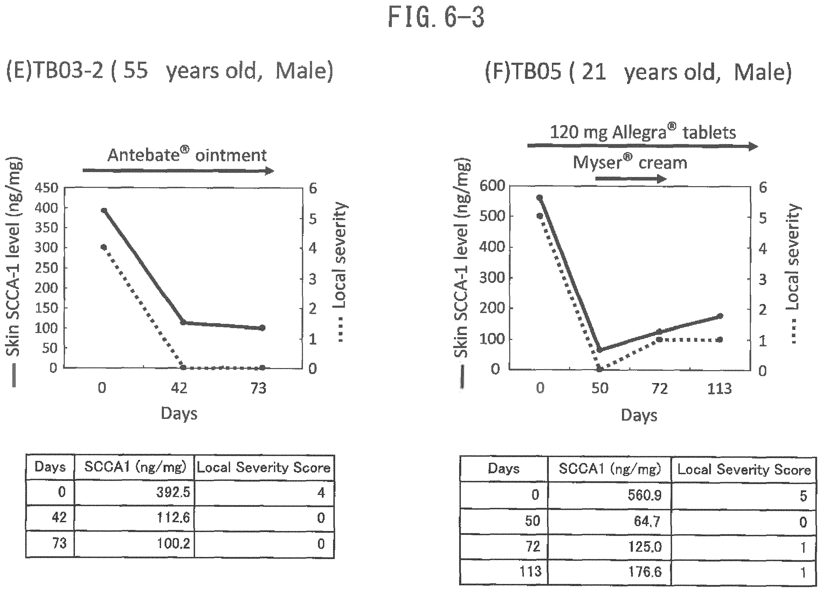

Test site 1 of patient TA04 exhibited a total skin finding score of 10 and severity was classified as moderate. During the treatment observation period, the patient was observed and samples of the corneal layer were collected at the time of each visit. The types of administered medications used by the patient, the administration period thereof, the names of administered topical preparations and the test sites where they were administered were confirmed. SCCA-1 level at the start of treatment was 6510 ng/mg, exhibiting a considerably high value. The same patient was continuously administered an antihistamine in the form of Allelock® OD and was given 100 mg Flomox® tablets. SCCA-1 level measured 28 days after the start of treatment was 415.9 ng/mg, indicating a remarkable decrease in comparison with the level at the start of treatment, and that result coincided with skin findings (total score: 1). The results are shown in Fig. 6(A).

### Monitoring of Treatment Progress 1-2

### Patient TB03

Skin findings of patient TB03 yielded a score of 4 and severity was classified as mild. During the treatment observation period, the patient was observed and samples of the corneal layer were collected at the time of each visit. The types of administered medications used by the patient, the administration period thereof, the names of administered topical preparations and the test sites where they were administered were confirmed. SCCA-1 level at the start of treatment was 392.5 ng/mg, exhibiting a value that was slightly higher than that of healthy subjects. The same patient was continuously administered topical steroid preparation in the form of Antebate® ointment. SCCA-1 level measured 42 days after the start of treatment was 112.6 ng/mg, indicating a remarkable decrease in comparison with the level at the start of treatment, and that result coincided with skin findings (total score: 0). The results are shown in Fig. 6(B).

### Monitoring of Treatment Progress 1-3

### Patient TA03

Skin findings of 28-year-old female patient TB03 yielded a total skin finding score of 7 and severity was classified as mild. During the treatment observation period, the patient was observed and samples of the corneal layer were collected at the time of each visit. The types of administered medications used by the patient, the administration period thereof, the names of administered topical preparations and the test sites where they were administered were confirmed. SCCA-1 level at the start of treatment was 1745.6 ng/mg, exhibiting a considerably high value. The same patient was continuously administered Antebate® ointment after 56 days. SCCA-1 level measured 56 days after the start of treatment was 39.6 ng/mg, indicating a remarkable decrease in comparison with the level at the start of treatment, and that result coincided with favorable skin findings (total score: 1). The results are shown in Fig. 6(C).

### Monitoring of Treatment Progress 1-4

### Patient TA04-2

Skin findings at test site 2 of 27-year-old male patient TA04 yielded a total score of 21 and severity was classified as severe. During the treatment observation period, the patient was observed and samples of the corneal layer were collected at the time of each visit. The types of administered medications used by the patient, the administration period thereof, the names of administered topical preparations and the test sites where they were administered were confirmed. SCCA-1 level at the start of treatment was 9119.9 ng/mg, exhibiting a considerably high value. The same patient was continuously administered an antihistamine in the form of Allelock® OD and was given 100 mg Flomox® tablets. SCCA-1 level measured 28 days after the start of treatment was 36.6 ng/mg, indicating a remarkable decrease in comparison with the level at the start of treatment, and that result coincided with favorable skin findings (total score: 1). The results are shown in Fig. 6(D).

### Monitoring of Treatment Progress 1-5

### Patient TB03-2

Skin findings at test site 2 of 55-year-old male patient TB03 yielded a total skin finding score of 4 and severity was classified as mild. During the treatment observation period, the patient was observed and samples of the corneal layer were collected at the time of each visit. The types of administered medications used by the patient, the administration period thereof, the names of administered topical preparations and the test sites where they were administered were confirmed. SCCA-1 level at the start of treatment demonstrated a value of 392.5 ng/mg. The same patient was continuously administered Antebate® ointment. SCCA-1 level measured 42 days after the start of treatment was 112.6 ng/mg, indicating a decrease in comparison with the level at the start of treatment, and that result coincided with favorable skin findings (total score: 0). The results are shown in Fig. 6(E).

### Monitoring of Treatment Progress 1-6

### Patient TB05

Skin findings of 21-year-old male patient TB05 yielded a total score of 5 and severity was classified as mild. During the treatment observation period, the patient was observed and samples of the corneal layer were collected at the time of each visit. The types of administered medications used by the patient, the administration period thereof, the names of administered topical preparations and the test sites where they were administered were confirmed. SCCA-1 level at the start of treatment demonstrated a value 560.9 ng/mg. The same patient was given an antihistamine in the form of 120 mg Allegra® tablets and continuously applied Myser® cream. SCCA-1 level measured 50 days after the start of treatment was 64.7 ng/mg, indicating a decrease in comparison with the level at the start of treatment, and that result coincided with extremely favorable skin findings (total score: 0). SCCA-1 levels measured 72 days and 113 days after the start of treatment increased to 125.0 ng/mg and 176.6 ng/mg, respectively, although the cause of this is unclear, skin findings also increased to a total score of 1 in comparison with the total score of 0 recorded 50 days after the start of treatment, again indicating that SCCA-1 levels and skin findings are linked. The results are shown in Fig. 6(F).

Based on the results of the treatment progress monitoring tests described above, SCCA-1 levels can be understood to be linked with severity of atopic dermatitis as determined by a physician. As has been described above, corneal layer SCCA-1 can be collected using an extremely easy and non-invasive procedure such as tape stripping. Since measurement of SCCA-1 can be performed not only at a hospital or testing facility but also at home by developing an easy assay kit, SCCA-1 is an extremely superior indicator for evaluating the severity of atopic dermatitis and the efficacy of current treatment. Since infants and small children account for the majority of atopic dermatitis patients in particular, being able to determine the severity of the disease on one's own at home by using SCCA-1 as an indicator is extremely advantageous for easily and objectively determining one's own severity at home and objectively evaluating treatment efficacy for patients who are unable to convey their symptoms on their own or for whom it is not easy to visit a hospital without assistance.

### 6. Observation of Progress

### Monitoring of Progress 2-1-1

### Patient AW1-1

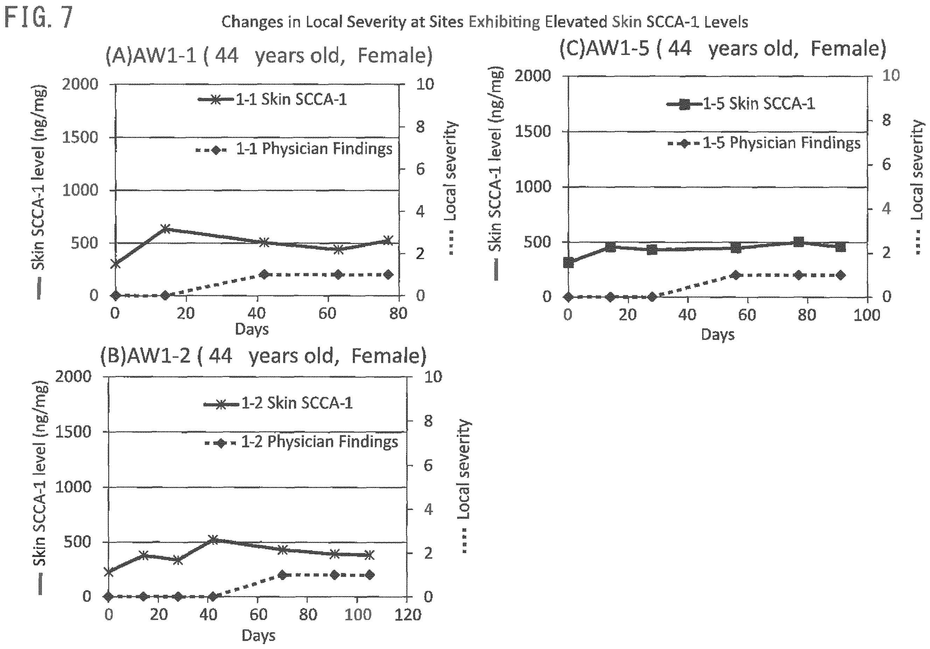

Test site no. 1 of patient AW1 was a site that was diagnosed as being in remission by a physician. The patient did not present with skin symptoms at the start of observation and skin findings as evaluated by a physician yielded a value (total score) of 0. During the treatment observation period, the patient was observed and samples of the corneal layer were collected at the time of each visit. Furthermore, this patient was not treated for atopic dermatitis during the observation period. SCCA-1 level at the start of treatment was about 302.4 ng/mg, indicating a level that was significantly higher than that of healthy subjects (19.1 ng/mg) and could therefore not be ignored. Although skin findings as determined by a physician yielded a score of 0 from the start of observation though about day 15, the skin findings score subsequently increased to 1 together with an increase in SCCA-1 level, indicating the beginning of exacerbation. Scores for self-determined findings as evaluated by the patient were 0, remaining unchanged throughout the observation period. The observation results are shown in Fig. 7(A).

### Monitoring of Treatment Progress 2-1-2

### Patient AW1-2

Test site no. 2 of patient AW1 was a site that was diagnosed by a physician as exhibiting mild severity at the start of treatment. The patient did not present with skin symptoms at the start of observation, and although skin findings as evaluated by a physician yielded a value (total score) of 0, findings as determined by a physician increased with the passage of time. Although self-determined findings increased slightly (from 0 to 1), self-determined scores were lower in comparison with those determined by a physician. Furthermore, this patient was not treated for atopic dermatitis during the observation period. SCCA-1 level at the start of treatment was about 227.0 ng/mg. Although skin findings as determined by a physician also yielded a score of 0 until around day 40 from the start of observation, the skin findings score subsequently increased to 1 together with an increase in SCCA-1 level, indicating the beginning of exacerbation. Furthermore, scores for self-determined findings as evaluated by the patient were 0, remaining unchanged throughout the observation period. The observation results are shown in Fig. 7(B).

### Monitoring of Treatment Progress 2-1-3

### Patient AW1-5

Test site no. 5 of patient AW1 was a site diagnosed to be in remission by a physician. The patient did not present with skin symptoms at the start of observation and skin findings as evaluated by a physician yielded a value (total score) of 0. During the treatment observation period, the patient was observed and samples of the corneal layer were collected at the time of each visit. Furthermore, this patient was not treated for atopic dermatitis during the observation period. SCCA-1 level at the start of treatment was about 313.4 ng/mg. Although skin findings as determined by a physician also yielded a score of 0 until around day 30 from the start of observation, the skin findings score subsequently increased to 1 together with an increase in SCCA-1 level, indicating the beginning of exacerbation. Furthermore, scores for self-determined findings as evaluated by the patient were 0, remaining unchanged throughout the observation period. The observation results are shown in Fig. 7(C).

As indicated by the above observation results, since disease demonstrated recurrence at test sites nos. 1, 2 and 5 in this patient, the patient was confirmed to be in remission at the start of observation. Since SCCA-1 levels exhibited values that could not be ignored at the start of the observation period, the use of SCCA-1 as an indicator makes it possible to determine whether or not a diagnosed site is in remission. In addition, SCCA-1 levels were linked with skin findings as determined by a physician.

### Monitoring of Treatment Progress 2-2-1

### Patient AW3-4

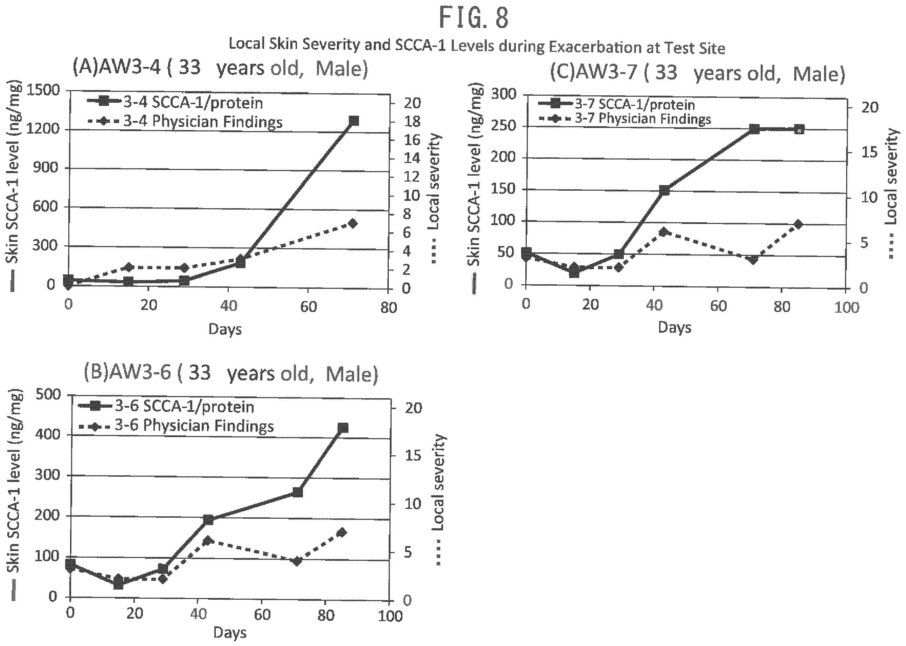

Test site no. 4 of patient AW3 did not present with skin symptoms even at the start of observation. During the observation period, the patient was observed and samples of the corneal layer were collected at the time of each visit. SCCA-1 level at the start of treatment was 43.9 ng/mg, which although was slightly low, was significantly higher in comparison with healthy subjects (19.1 ng/mg) and was high enough so that it could not be ignored. SCCA-1 level 43 days after the start of observation was 184.7 ng/mg, demonstrating a remarkable increase in comparison with the level at the start of observation, and that result coincided with skin findings determined by a physician (total score: 3). On the other hand, the increase in self-determining findings was only slight throughout the observation period (total score: 1). The results are shown in Fig. 8(A).

### Patient AW3-6

Test site no. 6 of patient AW3 did not present with skin symptoms even at the start of observation. During the observation period, the patient was observed and samples of the corneal layer were collected at the time of each visit. SCCA-1 level at the start of treatment was 82.5 ng/mg, which although was slightly low, was significantly higher in comparison with healthy subjects (19.1 ng/mg) and was high enough so that it could not be ignored. SCCA-1 level 85 days after the start of observation was 425.9 ng/mg, demonstrating a remarkable increase in comparison with the level at the start of observation, and that result coincided with skin findings determined by a physician (total score: 7). On the other hand, the increase in self-determining findings was only slight throughout the observation period (total score: 1). The results are shown in Fig. 8(B).

### Patient AW3-7

Test site no. 7 of patient AW3 did not present with skin symptoms even at the start of observation. During the observation period, the patient was observed and samples of the corneal layer were collected at the time of each visit. SCCA-1 level at the start of treatment was 50.7 ng/mg, which although was slightly low, was significantly higher in comparison with healthy subjects (19.1 ng/mg) and was high enough so that it could not be ignored. SCCA-1 level 85 days after the start of observation was 250.6 ng/mg, demonstrating a remarkable increase in comparison with the level at the start of observation, and that result coincided with skin findings determined by a physician (total score: 7). On the other hand, the increase in self-determining findings was only slight throughout the observation period (total score: 1). The results are shown in Fig. 8(C).

As indicated by the above observation results, since disease demonstrated recurrence at test sites nos. 4, 6 and 7 in this patient, the patient was confirmed to be in remission at the start of observation. Since SCCA-1 levels exhibited values that could not be ignored at the start of the observation period, the use of SCCA-1 as an indicator makes it possible to evaluate skin condition at those sites where symptoms are absent.

### 7. Correlation with SCORAD Index

The SCORAD index is frequently used to classify the severity of atopic dermatitis (NPL1). The area of eruptions (A) (ratio of the area of eruptions to body surface area) and the total score (B) for the degree of each eruption (0 = absent, 1 = mild, 2 = moderate and 3 = severe) of erythema, oozing/eruption, exudate/crusting, excoriation, lichenification and xerosis /dryness are determined and subjective symptoms as determined by the subject are evaluated according to VAS (C) followed by calculation of the SCORAD score using the formula A/5+7×B/2+C.

Corneal layer samples were collected by tape stripping from each subject for whom a SCORAD score was determined followed by measurement of SCCA-1 level. At that time, the subjects were divided into those who collected skin from 1 location only (N=1), those who collected skin from two locations (N=2) and those who collected skin from three locations (N=3), and the average values of SCCA-1 level were determined in the case of subjects who collected skin from two or three locations. Next, the correlation between the SCORAD scores of each subject and the SCCA-1 levels of each subject in the N=1, N=2 and N=3 groups was determined. The following provides a summary of the results.

**[Table 2]**

| Number of Test Sites | Spearman's Correlation Coefficient | P-value | Number of subjects (n) |
|---|---|---|---|
| N=1 | 0.337 | 0.0683 | 30 |
| N=2 | 0.427 | 0.1667 | 12 |
| N=3 | 0.587 | 0.0132* | 17 |

On the basis of the above results, it was found that when skin was collected from at three locations and the average SCCA-1 level thereof was determined, the average SCCA-1 level was highly correlated with SCORAD score. The SCORAD index has the shortcomings of requiring specialized work performed by an experienced physician to calculate SCORAD scores while also taking considerable time. Thus, a method is provided that enables both simple and objective classification of severity by using corneal layer SCCA-1 level as an indicator as an alternative to SCORAD.

### INDUSTRIAL APPLICABILITY

The present invention makes it possible to provide a diagnosis assisting method for simple and objective evaluation of a treatment strategy of atopic dermatitis in a subject undergoing the treatment thereof, by using SCCA-1 in corneocytes as an indicator.

## Claims

1. A diagnosis assisting method for determining a treatment strategy of atopic dermatitis in a subject undergoing the treatment thereof,
wherein the method comprises:
measuring the SCCA-1 expression level in corneocytes of the subject; and
i) if the SCCA-1 expression level of the subject is not statistically significantly higher than the SCCA-1 expression level of those who do not have atopic dermatitis, discontinuing the treatment and determining that there is no need of continuing the treatment.

2. A diagnosis assisting method for determining a treatment strategy of atopic dermatitis in a subject undergoing the treatment thereof,
wherein the method comprises:
measuring the SCCA-1 expression level in corneocytes of the subject; and
ii) classifying the severity of atopic dermatitis of the subject by using the SCCA-1 expression level as an indicator, and determining to apply a treatment method for atopic dermatitis corresponding to respective class to the subject.

3. A diagnosis assisting method for determining the necessity of the treatment of atopic dermatitis in a subject in need of such a determination or a subject undergoing the treatment thereof, or for determining a treatment strategy of atopic dermatitis if it is determined that the treatment thereof is necessary,
wherein the method comprises:
measuring the SCCA-1 expression level in corneocytes of the subject; and
i) if the SCCA-1 expression level of the subject is not statistically significantly higher than the SCCA-1 expression level of those who do not have atopic dermatitis, determining that there is no need of conducting the treatment or continuing the treatment if the subject is undergoing the treatment; or
if the SCCA-1 expression level of the subject is statistically significantly higher than the SCCA-1 expression level of those who do not have atopic dermatitis, determining that there is a need of conducting the treatment or continuing the treatment if the subject is undergoing the treatment, and furthermore
ii) classifying the severity of atopic dermatitis of the subject by using the SCCA-1 expression level of the subject as an indicator, and determining to apply a treatment method for atopic dermatitis corresponding to respective class to the subject.

4. The diagnosis assisting method according to any one of claims 1 to 3, wherein said determination of the treatment strategy is repeatedly performed over a long term.

5. The diagnosis assisting method according to any one of claims 1 to 4, wherein said severity of atopic dermatitis is regarding one or more items selected from the group consisting of erythema, oozing, papulation, excoriation, lichenification, xerosis/dryness and itch.

6. The diagnosis assisting method according to any one of claims 1 to 5, wherein said skin corneocytes are collected in a non-invasive manner by tape stripping.

7. The diagnosis assisting method according to any one of claims 1 to 6, wherein said measurement of the SCCA-1 expression level is performed by immunoassay using an SCCA-1 specific antibody.

8. The diagnosis assisting method according to claim 7, wherein said immunoassay is selected from the group consisting of enzyme immunoassay (including ELISA, EIA), fluorescent immunoassay, radioimmunoassay (RIA method), luminescent immunoassay, surface plasmon resonance (SPR), quartz crystal microbalance (QCM), immune turbidimetry, latex agglutination immunoassay, latex turbidimetry, particle agglutination reaction method, gold colloid method, capillary electrophoresis, graphene biosensor, amperometric biosensor, laser spectrometry, and surface enrichment immunoassay.

9. The diagnosis assisting method according to any one of claims 6 to 8, wherein said collection of skin corneocytes and said measurement of the SCCA-1 expression level is conducted at home or medical checkup.

10. The diagnosis assisting method according to any one of claims 1 to 9, wherein said collection by tape stripping is performed on three or more sites of the skin of the subject.
